# EUROPÄISCHE PATENTSCHRIFT

(11) **EP 2 644 210 B1**
(45) Veröffentlichungstag und Bekanntmachung des Hinweises auf die Patenterteilung: **03.01.2018**
(21) Anmeldenummer: 13155570.8
(22) Anmeldetag: 18.02.2013
(51) Int. Cl.: A61L 29/04, A61M 25/00

(54) **Katheterschaft mit verschweißten Schläuchen**
Catheter shaft comprising welded tubes
Tige de cathéter avec tubulures soudées

(30) Priorität: 29.03.2012 US 201261617059 P
(43) Veröffentlichungstag der Anmeldung: 02.10.2013
(73) Patentinhaber: Biotronik AG, 8180 Bülach (CH)
(72) Erfinder: Schwitzer, Alwin, 8180 Bülach (CH); Lang, Hans, 8107 Buchs (CH)
(74) Vertreter: Randoll, Sören

(56) Entgegenhaltungen:
- US-A1- 2010 063 476

## Beschreibung

Die Erfindung betrifft einen Katheterschaft, umfassend einen ersten (inneren und/oder äußeren) Schlauch aus einem extrudierbaren fluorierten Copolymer und einen mit dem ersten Schlauch verschweißten zweiten Schlauch aus einem polymeren, bevorzugt thermoplastischen, Material.

Die Erfindung betrifft ferner ein Medizinprodukt, umfassend einen solchen Katheterschaft, sowie die Verwendung eines ersten Schlauchs aus einem extrudierbaren fluorierten Copolymer zum Verschweißen mit einem zweiten polymeren Schlauch. Die Erfindung betrifft außerdem ein Verfahren zum Herstellen eines Katheterschaftes oder eines Medizinproduktes mit den entsprechenden Schläuchen sowie einen bestimmten, besonders geeigneten Schlauch.

Im Markt werden zurzeit mehrschichtige Schläuche für den Innenschaft eines Katheters verwendet. Dazu gehören z.B. mehrschichtige Polytetrafluorethylen(PTFE)-Schläuche, coextrudierte HDPE(high density Polyethylen)-Schläuche und Ähnliches mit einer Polyamid-basierenden Außenschicht, um diese Innenschäfte mit anderen Schläuchen, die eine zweite Schlauchschicht aus einem polymeren thermoplastischen Material enthalten, verschweißen zu können.

Fluorierte Polymere (z.B. PTFE) weisen im Allgemeinen die geringsten Reibungskoeffizienten gegenüber anderen Materialien aus, zeigen eine hohe Alterungsbeständigkeit und sind sehr chemikalienresistent. Nachteile von PTFE sind der aufwändige Herstellungsprozess von Schläuchen (nicht durch herkömmliche Extrusion, da PTFE nicht thermoplastisch verarbeitet werden kann, da PTFE nicht schmelzbar ist) und die geringe Abriebfestigkeit. Ein Nachteil der PTFE-basierenden Innenschäfte sind zudem der hohe Preis und die Abhängigkeit von bestimmten Herstellern. Ein weiterer Nachteil von PTFE als Material für Medizinprodukte ist, dass PTFE nicht durch Strahlensterilisationsverfahren sterilisiert werden kann.

Andere thermisch verformbare (extrudierbare) fluorierte Polymere mit ebenfalls geringen Reibungskoeffizienten (z B. ETFE (Polytetrafluorethylen + Ethylen, E-CTFE (Polychlortrifluorethylen + Ethylen), PFA (Polytetrafluorethylen + Perfluorpropylether), FEP (Polytetrafluorethylen + Perfluorpropylen), PCTFE (Polychlortrifluorehtylen), PVF (Polyvinylfluorid) und PVDF (Polyvinylidenfluorid)) können mit den etablierten Katheter-Polymeren (wie z.B. Polyamid 12 oder PEBA), die z.B. für Außenschäfte, Spitzen und Ballone verwendet werden, nicht verschweißt und aufgrund der geringen Oberflächenspannung und der dadurch reduzierten Benetzbarkeit auch nur schlecht verklebt werden.

Deshalb haben sich für die Innenschäfte für z.B. RX-Katheter (mit reduzierter Reibung zu Führungsdrähten) nur sehr aufwändige Mehrschicht-Schlauch-Strukturen etabliert. Unter anderem bekannt sind Schläuche mit PTFE als Innenschicht und einer polyamidbasierenden Außenschicht oder Mehrschichtstrukturen mit einer HDPE-Innenschicht und ebenfalls einer polyamidbasierenden Außenschicht.

Der Nachteil von coextrudierten, z.B. HDPE-basierenden, Innenschaftkonstruktionen ist auch der teure Herstellungsprozess durch eine Coextrusion von wenigstens zwei, üblicherweise drei, Schichten mit jeweils einem Extruder für jede Schicht.

Die US 2010/0063476 A1 beschreibt die Verwendung von modifiziertem PVDF als Innenschicht von coextrudierten Schläuchen. Eine Coextrusion ist apparativ sehr aufwändig und verlangt Arbeiten mit mindestens zwei, üblicherweise drei separaten Extrudern, die aufeinander abgestimmt werden müssen, damit die zwei oder drei coextrudierten Schichten die gewünschten Schichtdickenverteilungen bei den finalen Schlauchdimensionen aufweisen. Dabei müssen die Temperaturen der separaten Extruder so gewählt werden, dass die unterschiedlichen Schlauchschichten bei der Extrusion miteinander verbunden werden, was üblicherweise bei einer 3-Schichtextrusion über eine physikalische Anbindung zwischen der HDPE-Innenschicht zur Mittelschicht und über eine chemische Anbindung der Mittelschicht an die polyamidbasierende Außenschicht erfolgt. Dementsprechend sind die Anforderungen Druck und Temperatur hoch. Allerdings führt die Coextrusion zu einer kovalenten Bindung der beiden Schlauchschichten, was grundsätzlich erwünscht ist.

Die Dokumente AU 2004/202463 B2 und EP 1 484 346 sowie US 2010/0255378 A1 offenbaren jeweils Copolymere von Fluorpolymeren (modifizierte Fluorpolymere).

Es war Aufgabe der Erfindung, weniger aufwändig herstellbare Katheterschäfte anzugeben, die bevorzugt einen möglichst geringen Reibungskoeffizienten, bevorzugt hohe Chemikalienbeständigkeit und/oder hohe Altersbeständigkeit besitzen sollten. Außerdem sollten diese Schäfte mit üblichen polymerbasierten Katheterbauteilen, insbesondere auf Polyamid basierten Bauteilen, verschweißbar sein. Als weitere bevorzugte Eigenschaft sollten die Bestandteile der Katheterschäfte effektiv strahlensterilisierbar sein.

Diese Aufgabe wird gelöst durch einen Katheterschaft, umfassend (i) einen ersten (inneren und/oder äußeren) Schlauch aus einem extrudierbaren fluorierten Copolymer, das reaktive Gruppen umfasst und (ii) einen mit dem ersten Schlauch verschweißten zweiten Schlauch aus einem polymeren Material, wobei das polymere Material des zweiten Schlauchs bei einer Temperatur von ≤ 200°C mit dem ersten Schlauch verschweißbar ist.

Reaktive Gruppen sind solche Molekülgruppen, die eine höhere Reaktivität gegenüber Nucleophilen haben als Methyl- oder Methylengruppen. Bevorzugt im Sinne der Erfindung zeigen reaktive Gruppen wenigstens die Reaktivität einer Carbonylgruppe, besonders bevorzugt einer Carbonsäurederivatgruppe, ganz besonders bevorzugt einer Carbonsäureanhydridgruppe, gegenüber nucleophilen Agenzien, bevorzugt gegenüber einer Hydroxy- und/oder Amingruppe. Ein Copolymer im Sinne der vorliegenden Erfindung kann bereits dann vorliegen, wenn an ein klassisches Polymergrundgerüst lediglich reaktive Gruppen eingefügt sind. Es besteht aber grundsätzlich die Möglichkeit, dass reaktivere Gruppen tragende Seitenketten an das Polymergrundgerüst gebunden sind, bevorzugt sind sie gepfropft.

Verschweißen im Sinne der vorliegenden Erfindung bedeutet, dass zwei Schichten (hier insbesondere die Außenschicht eines und die Innenschicht eines anderen Schlauches), die sich im Wesentlichen im festen Zustand befinden durch (relativ kurze) Hitzeeinwirkung kovalent verbunden werden. Hierbei ist im Sinne der Erfindung nicht ausgeschlossen, dass im Bereich der Grenzflächen der beiden Schichten ein (kurzzeitiges) Erweichen durch Anschmelzen eintritt.

Verschweißt im Sinne der vorliegenden Erfindung bedeutet, dass wenigstens eine punktuelle Verschweißung vorliegt, d.h. dass der erste und zweite Schlauch im Sinne der vorliegenden Erfindung nicht flächig miteinander verschweißt sein müssen, sondern punktuelle Schweißverbindungen erfindungsgemäß ausreichen. Bevorzugt sind jedoch Schweißverbindungen, die wenigstens den gesamten Umfang im Bereich eines Schlauchabschnittes (also eines Teils der longitudinalen Ausdehnung des Schlauches) miteinander verbinden. Dies bedeutet, dass streng genommen das Lumen des äußeren, an den inneren Schlauch verschweißten, Schlauches nicht mehr durchgängig sein muss.

Extrudierbar im Sinne der vorliegenden Erfindung bedeutet, dass das (getrocknete) Material (bevorzugt ein thermoplastisch formbares Polymer oder Copolymer) in einem Einschneckenextruder mit einem Durchmesser von 12 bis 30 mm und einem Längen- zu Durchmesser-Verhältnis (L/D) von 24 bis 28 bei Temperaturen, die 50°C bis 100°C über dem Schmelzpunkt des thermoplastischen Polymers liegen, aufgeschmolzen werden kann, die Polymerschmelze zu einem Formgebungsteil (Extrusionskopf), bestehend aus einer Düse, die die äußere Schlauchschicht und einem Dorn, der die innere Schlauchschicht formt, gefördert werden und durch "Abschrecken" der geformten Polymerschmelze in einem Wasserbad in eine bleibende Form gebracht werden kann.

Es hat sich gezeigt, dass geeignete modifizierte fluorierte Polymere (Copolymere) in der Lage sind, sich mit einer Vielzahl von insbesondere thermoplastischen polymeren Materialien, die bevorzugt nucleophile Gruppen aufweisen, mit einer ausreichenden Festigkeit zu verbinden. Im Stand der Technik wurden solche Verbindungen aber regelmäßig durch Coextrusion erzeugt. Es hat sich aber überaschenderweise herausgestellt, dass es möglich ist, bei Temperaturen von ≤ 200°C einen bereits verfestigen Schlauch aus einem fluorierten Copolymer, das reaktive Gruppen umfasst, mit einer Vielzahl von geeigneten zweiten separaten Schläuchen zu verschweißen. Bislang ging man davon aus, dass die entsprechenden Materialien nur über Coextrusion ausreichend miteinander verbunden werden können.

Bevorzugt ist, dass zumindest der erste Schlauch, aber weiter bevorzugt beide Schläuche, beim Verschweißen jeweils als eigene Schläuche vorliegen. Bevorzugt ist ein möglichst großflächiges Verschweißen, das bedeutet, dass die beiden Schläuche unterschiedliche Durchmesser haben, so dass der eine Schlauch in den anderen geschoben werden kann. Selbstverständlich müssen Innendurchmesser und Wandstärken der Schläuche in geeigneter Weise aneinander angepasst sein. Bevorzugt ist eine flächige Verschweißung entlang der außenumlaufenden Fläche (des Innenschlauches) bzw. der innenumlaufenden Fläche des Außenschlauches.

Dabei umfasst die Erfindung sowohl die Ausgestaltung, dass der erste Schlauch der äußere Schlauch ist als auch die Ausgestaltung, dass der erste Schlauch der innere ist. Weitere Schichten und/oder Schläuche sind nicht ausgeschlossen, es ist aber bevorzugt, dass der Katheterschaft lediglich aus dem ersten und dem zweiten Schlauch besteht.

Auf Basis der vorliegenden Anmeldung ist es dem Fachmann möglich, Materialien und die Verschweißflächengrößen so anzupassen, dass eine gute Haltbarkeit der Schweißstellen besteht. Bevorzugt ist aber im Sinne der Erfindung, dass diese Verschweißungen so ausgeführt sind, dass sie einem Ballondruck von 20 bar, bevorzugt 24 bar, für ≥ 1 min, bevorzugt ≥ 2 min, besonders bevorzugt ≥ 10 min, unverändert standhalten. Selbstverständlich wird der Fachmann hierfür auch die Gesamtgeometrie der Schweißflächen berücksichtigen.

Erfindungsgemäß ist ein Katheterschaft bevorzugt, wobei das fluorierte Copolymer ein thermoplastisches Copolymer ist auf Basis von einem Polymer, ausgewählt aus der Gruppe bestehend aus ETFE, C-ETFE, PFA, FEP, PCTFE, PVF und PVDF.

Besonders bevorzugt ist in diesem Zusammenhang ein modifiziertes, bevorzugt mittels Propfung von Maleinsäureanhydrid verändertes, PVDF.

Die genannten Materialien eignen sich besonders gut aufgrund ihrer chemischen Widerstandsfähigkeit und wegen ihres geringen Reibwertes. Die genannten Copolymere können als erster Schlauch extrudiert werden und sowohl als innerer als auch als äußerer Schlauch eingesetzt werden, je nach Anwendungszweck des Katheterschaftes bzw. des diesen Katheterschaft umfassenden Medizinproduktes.

Generell sind neben den explizit genannten fluorierten Polymeren thermisch verformbare fluorierte Polymere für den ersten Schlauch (in einer entsprechenden Modifikation) bevorzugt.

Bevorzugte reaktive Gruppen im fluorierten Copolymer für die Anbindung an den zweiten Schlauch sind dabei in die Gruppen (chemischen Funktionen) ausgewählt aus der Gruppe bestehend aus Carbonsäure, Carbonsäurechlorid, Amid, Carbonsäureanhydrid, Ester, Lacton, Lactam, Nitril und Thioester.

Mittels dieser reaktiven Gruppen sind nucleophile Angriffe des Polymermaterials des zweiten Schlauchs gut gewährleistet, so dass eine Vielzahl von Materialkombinationen möglich ist.

Im Sinne der vorliegenden Erfindung ist es bevorzugt, dass der zweite Schlauch ausgewählt ist aus der Gruppe Polyamid, PEBA (Polyetherblockamid), Polyester, thermoplastische Polyurethanelastomere (TPU).

Generell sind für den zweiten Schlauch thermoplastische Polymere bevorzugt.

Durch die Modifikation der fluorierten Polymere lässt sich, wie oben angedeutet, eine zuverlässige Verbindung zu den Polymeren des zweiten Schlauchs herstellen. Überraschend in diesem Zusammenhang war jedoch, wie ebenfalls oben angedeutet, dass unter verhältnismäßig milden Bedingungen auch eine Verschweißung möglich und nicht zwingend eine Coextrusion erforderlich ist.

Somit werden durch die Erfindung weitere Materialkombinationen zugänglich, so dass sich dem Fachmann gegenüber dem Stand der Technik weitere Möglichkeiten ergeben, den Katheterschaft oder diesen umfassende Medizinprodukte an die jeweiligen Anforderungen anzupassen. So ist z.B. möglich, die Innenschäfte für RX-Katheter (mit reduzierter Reibung zu Führungsdrähten) auszugestalten, ohne dass aufwändige Mehrschichtschlauchstrukturen mit Haftvermittlungszwischenschichten erforderlich sind. Es besteht zudem eine gute Alternative zu PTFE als Innenschicht und es bestehen weiterhin Alternativen zu den marktüblichen HDPE-Innenschichten.

Auch die mechanischen Eigenschaften des Gesamtsystems können durch die erfindungsgemäße Kombination verbessert werden.

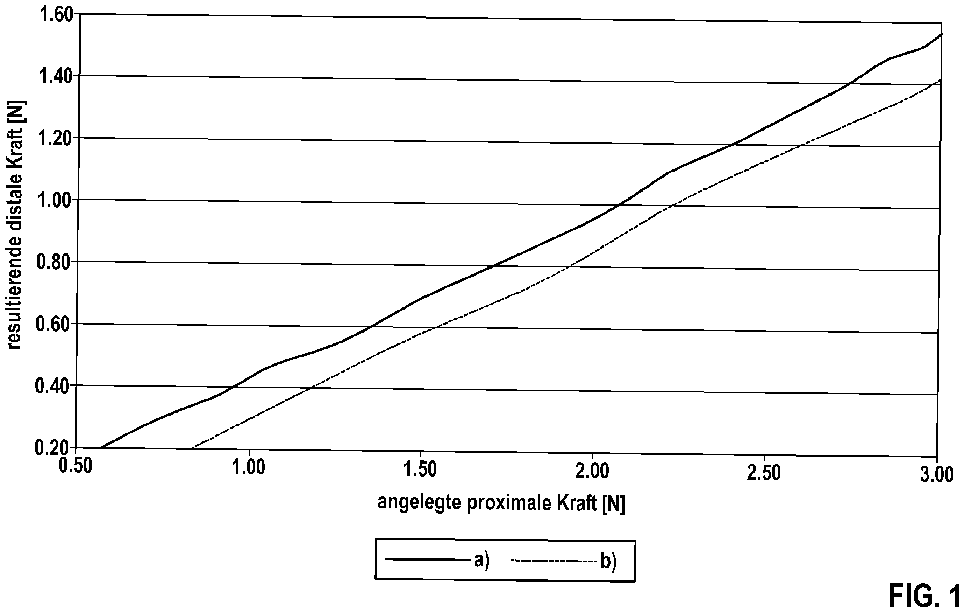

- Fig. 1: stellt die Pushability (Kraftübertragung) an coronaren Ballondilatationskathetern mit
a) Ballondilatationskatheter mit erfindungsgemäßem Innenschaft
b) Ballondilatationskatheter mit einem coextrudierten Innenschaft mit HDPE als Innenschicht nach dem Stand der Technik dar. Die Figur zeigt somit einen Vergleich der mechanischen Kräfte, die beim Vorschub eines erfindungsgemäßen Katheterschaftes eine Rolle spielen, verglichen mit einem Schaft aus dem Stand der Technik.

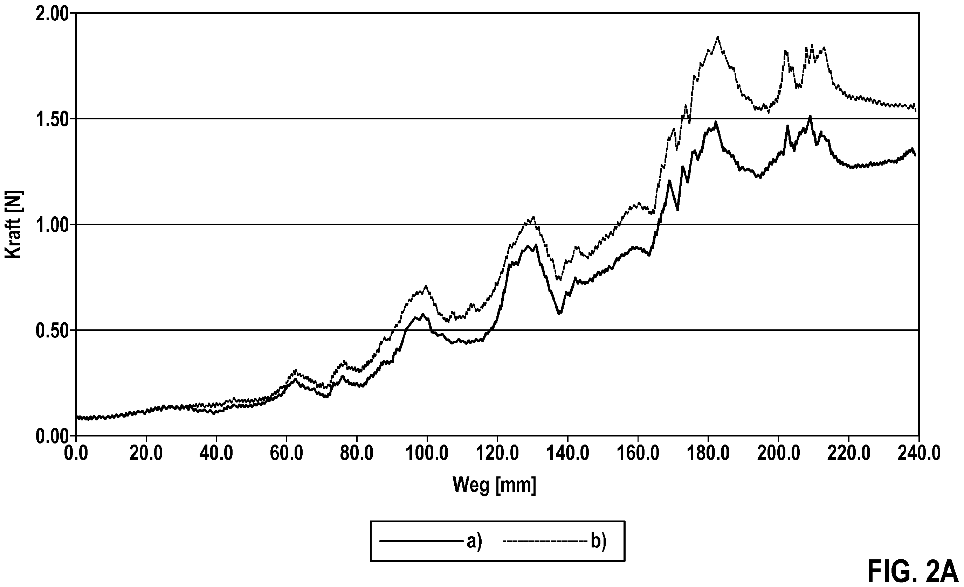

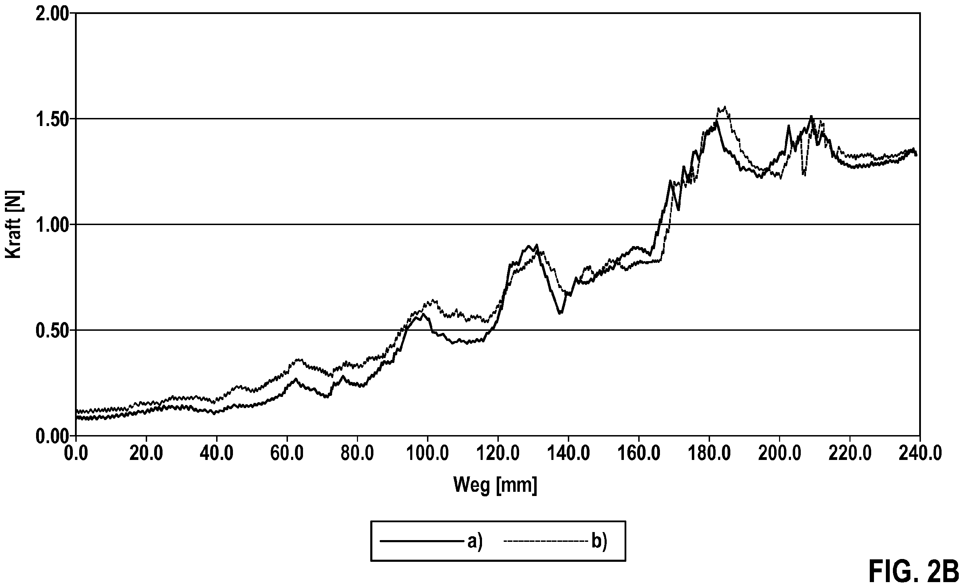

- Fig. 2 a und 2b: stellen die Trackability (Reibungskräfte) an coronaren Ballondilatationskathetern in einem Aorten-Modell mit Teflon®-Schlauch als Reibungspartner dar.
Führungsdraht: Galeo M "014; Führungskatheter: Cordis Vista 5F JL 4LBT
a) Ballondilatationskatheter mit erfindungsgemäßem Innenschaft
b) Ballondilatationskatheter mit einem Coextrudierten Innenschaft (US 2010/0063476 A1
Somit zeigen die Figuren 2a und 2b entsprechende Daten für die Reibung.

Ein erfindungsgemäßer (Ballondilatations)Katheter zeigt im Vergleich zu einem Ballondilatationskatheter mit einem coextrudierten HDPE-Innenschaft eine höhere Übertragung der am proximalen Ende angelegten Kraft auf das distale Ende des Katheters (Fig. 1).

Ein Vergleich der Reibungskräfte eines erfindungsgemäßen Ballondilationskatheters gegen die eines Ballondilationskatheters gemäß US 2010/0063476 A1 ist in Fig. 2a dargestellt. Beide Ballondilatationskatheter sind - bis auf die unterschiedlichen Innenschäfte - identisch. Bei diesem Test werden die Katheter in vitro in einem Modell, welches einer Simulation einer Koronararterie entspricht, eingeführt und die benötigte proximale Kraft bestimmt. Der erfindungsgemäße Ballondilatationskatheter zeigt gegenüber dem Katheter nach US 2010/0063476 A1 deutlich geringere Kräfte, was auf eine niedrige Reibung bei dem erfindungsgemäßen Innenschaft gegenüber dem Katheter nach US 2010/0063476 A1 hindeutet. Auch gegenüber einem Katheter, der einen coextrudierten HDPE-Innenschaft nach dem Stand der Technik hat, zeigt der erfindungsgemäße Ballondilatationskatheter leicht verbesserte Reibungskräfte (Fig. 2b).

Entsprechend den vorzitierten Untersuchungen ist erfindungsgemäß besonders bevorzugt ein erster Schlauch für einen einschichtigen Katheterschaft aus einem mit Maleinsäureanhydrid gepfropften PVDF, welches in einem konventionellen Einschneckenextruder (Ø 12 - 30 mm / L/D 24-28) aufgeschmolzen und über ein Formgebungsteil zu einem Schlauch geformt wird. Die geformte Schmelze wird in einem Wasserbad abgeschreckt. Der zweite Schlauch, der mit dem ersten Schlauch verschweißbar ist, besteht aus einer Polyamidschicht, bevorzugt aus einem (zunächst) separaten Schlauch.

Ein besonders bevorzugt einzusetzendes fluoriertes Copolymer ist das Material mit dem Handelsnamen "Kynar ADX" der Firma Arkema. Aus diesem Material können kostengünstig durch Extrusion Schläuche hergestellt werden.

Kynar ADX wurde von Arkema für die Coextrusion von PVDF mit Polyamid 12, z.B. für Automobil-Kraftstoffleitungen, entwickelt. Kynar ADX ist ein funktionalisiertes Polyvinylidenfluorid, welches eine chemische Bindung zu thermoplastischen Polymeren, z.B. Polyamid, PEBA, Polyester, TPU etc., eingehen kann. Die reaktiven funktionellen Gruppen aus Maleinsäureanhydrid werden durch Compoundierung und leichte Strahlenvernetzung in das ursprüngliche PVDF eingeführt. In US 2010/0063476 A1 wird dieses Material für die Coextrusion von einem zweischichtigen Schlauch verwendet - entsprechend dem Anwendungsgebiet des Herstellers.

Die chemische Modifikation von Kynar ADX ist konzipiert für die Entstehung einer chemischen Verbindung beim Zusammentreffen von zwei Polymerschmelzen im z.B. Coextrusionsprozess, bei der das Maleinsäureanhydrid des Kynar ADX über nucleophile Gruppen der zweiten Schicht angegriffen wird, wobei mindestens eine Amidbindung, vorzugsweise eine Imidbindung, entsteht.

Überraschenderweise können Schläuche aus Kynar ADX auch nach einer durchgeführten thermischen Behandlung eine chemische Verbindung zu anderen polyamidbasierten Bauteilen (Verschweißung) eingehen, d.h. die chemische Modifikation funktioniert auch nach der Extrusion und dem damit verbundenen Eintauchen der geformten Kynar-ADX-Schmelze in Wasser. Dies wurde für dieses Material bisher nicht beschrieben, da die Maleinsäureanhydridgruppen an der Schlauchoberfläche, die Kontakt zum Wasser haben, zu weniger reaktiven Dicarbonsäuregruppen verseift werden. Durch leichtes Anschmelzen der Oberflächen der beiden zu verschweißenden Fügepartner (hier erster und zweiter Schlauch) wird eine kovalente chemische Bindung zwischen den beiden Fügepartnern erzeugt. Diese ist ausreichend, um die verschiedenen Bauteile miteinander zu verschweißen.

Gegenüber PTFE weist PVDF außerdem eine bessere Strahlensterilisierbarkeit auf (maximale Strahlendosis PVDF 1500kGy, Teflon < 20kGy).

Diese erfindungsgemäßen ersten Schläuche aus modifiziertem PVDF können z.B. als Innenschaft für RX-Katheter verwendet werden. Sie können proximal mit einem polyamidbasierten Außenschaft und distal mit einem polyamidbasierten Ballon und/oder Spitzen, insbesondere mittels Laser oder thermischer Energie, chemisch verschweißt werden.

Alternativ besteht auch die Möglichkeit, den entsprechenden ersten Schlauch als Außenschlauch für RX-Katheter ohne Coating oder als Außenschlauch für selbstexpandierbare Stent Delivery Devices zu verwenden. Hier wird insbesondere nach außen hin die geringe Reibung des Materials ausgenutzt. Somit sind die besonders hervorzuhebenden Vorteile der erfindungsgemäßen Katheterschäfte folgende:
- Es ist eine einfache Extrusion des ersten Schlauchs ohne Coextrusion möglich. Dies bedeutet eine erhebliche Kostenersparnis, insbesondere hinsichtlich der Extrusionsvorrichtungen und -bedingungen.
- Die ersten Schläuche (insbesondere in den bevorzugten Formen) zeigen sowohl als Außen- als auch als Innenschlauch innerhalb eines Katheterschaftes eine deutlich verbesserte (verringerte) Reibung.
- Es bestehen verbesserte mechanische Eigenschaften hinsichtlich der Einführbarkeit.
- Eine zuverlässige Strahlensterilisierbarkeit ist möglich.
- Eine Verschweißung des ersten Schlauchs aus dem modifizierten PVDF ist zu polyamidbasierenden zweiten Schläuchen über eine chemische Anbindung möglich.

Ferner ist es möglich, ohne Schwierigkeiten weitere Bestandteile an den Katheterschaft anzuschweißen.

Dementsprechend ist ein Teil der Erfindung ein Medizinprodukt, umfassend einen Katheterschaft, wie oben beschrieben.

Besonders bevorzugt ist ein solches Medizinprodukt ausgewählt aus der Gruppe bestehend aus Katheterstenteinlegungsvorrichtung oder Ballondilationskatheter, insbesondere in RX-(Rapid Exchange) oder "Over the Wire"-Ausführung.

Teil der Erfindung ist auch ein (erster) Schlauch aus einem mit Maleinsäureanhydrid als reaktiver Gruppe gepfropften PVDF, welches insbesondere in einem konventionellen Einschneckenextruder (Ø 12 - 30 mm / L/D 24-28) aufgeschmolzen und über ein Formgebungsteil zu einem Schlauch geformt wurde und der insbesondere mit einem zweiten polyamidbasierenden Schlauch, einem separaten Schlauch verschweißbar ist, als Bestandteil eines erfindungsgemäßen Katheterschaftes oder eines erfindungsgemäßen Medizinproduktes.

Bei dem besonders bevorzugten erfindungsgemäß einzusetzenden Material gehen die Erfinder davon aus - ohne an die Theorie gebunden zu sein -, dass die reaktive Gruppe (Maleinsäureanhydrid) nach der Extrusion im Wasser hydratisiert wird, so dass - anders als vom Hersteller angegeben - auch bei einer Verschweißung bei ≤ 200°C eine chemische Reaktion mit beispielsweise Aminogruppen möglich ist. Bislang wurde davon ausgegangen, dass für eine zuverlässige Verbindung des Materials mit einem entsprechenden zweiten Schlauch Temperaturen von > 220°C und Drücke im Bereich von 200 bar erforderlich sind. Unter diesen Bedingungen reagiert das Maleinsäureanhydrid mit Aminogruppen eines Polyamids und bildet chemische Imidverbindungen.

Beim Verschweißen, wie erfindungsgemäß vorgesehen, reagiert dagegen eine Carbonsäurefunktion mit dem Amin zu einer Amidgruppe. Dementsprechend ist nur ein leichtes Anschmelzen beim Verschweißen (T ≤ 200°C) erforderlich, wobei bevorzugt ist, dass grundsätzlich beim Schweißen ein Schrumpfschlauch eingesetzt wird, um die beiden Schläuche aneinanderzupressen.

Im Vergleich mit dem Stand der Technik wirkt sich das wie folgt aus:

Bei der 3-Schicht-Coextrusion von HDPE/Haftvermittler/Polyamid-Innenschäften werden die 3 verschiedenen Materialien in 3 verschiedenen Extrudern bei unterschiedlichen Temperaturen aufgeschmolzen. Die 3 Polymerschmelzen werden zu einem Formgebungsteil (Coextrusionskopf) geführt, wo die 3 Polymerschmelzen aufeinandertreffen. Dabei findet zwischen der HDPE und der Haftvermittlerschicht eine physikalische Anbindung und zwischen der Polyamidschicht und der Haftvermittlerschicht eine chemische Anbindung statt. Die HDPE-Innenschicht dient bei dieser Konstruktion für eine geringe Reibung gegenüber dem Führungsdraht. Die Haftvermittlerschicht verbindet die HDPE-Innenschicht mit der Polyamid-Außenschicht. Die Polyamid-Außenschicht dient zur Verbindung mit polyamidbasierenden anderen Schläuchen, Spitzen oder Ballon (= Schweißpartner). Dieselbe Wirkungsweise findet auch bei der Coextrusion des Innenschaftes in der Offenlegungsschrift US 20120/0063476 A1 statt. In diesem Fall besteht die Innenschicht aus dem fluorierten modifizierten Polymeren PVDF, welches über die chemische Modifikation bei der Coextrusion mit der polyamidbasierenden Außenschicht verbunden wird. Die PVDF-Innenschicht dient zur Reduzierung der Reibung zum Führungsdraht und die polyamidbasierende Außenschicht dient zur Verschweißbarkeit zu weiteren polyamidbasierenden Schläuchen, Ballonen oder Spitzen. Dabei entsteht eine physikalische Verschweißung der beiden Partner, bei der durch das Anschmelzen der beiden zu verbindenden Oberflächen eine physikalische Durchmischung stattfindet.

Bei der erfindungsgemäßen Extrusion des modifizierten PVDF wird nur ein einschichtiger Schlauch extrudiert, d.h. die Innen- und Außenoberfläche des Schlauches besteht aus dem modifizierten PVDF. Die Verschweißung mit weiteren Katheterbauteilen (z.B. weiteren Schläuchen, Ballonen oder Spitzen) erfolgt über die chemische Reaktion der enthaltenen Maleinsäureanhydrid- oder der gebildeten Maleinsäuregruppen mit nucleophilen Guppen, die in den Polymeren der separaten Schweißpartner enthalten sind. Die beiden zu verbindenden Oberflächen sind demnach chemisch über Amid- resp. Imidbindungen miteinander verbunden und zeigen keine Durchmischung.

Demnach unterscheiden sich die Schweißpartner bei der konventionellen Innenschaftkonstruktion mit einer physikalischen Durchmischung der beiden Oberflächenmaterialien grundsätzlich von der erfindungsgemäßen chemischen Verschweißung der beiden Oberflächenmaterialien (ohne Durchmischung der beiden Materialien).

Wie bereits oben beschrieben weisen die erfindungsgemäßen Katheterschäfte - und damit auch erfindungsgemäße Medizinprodukte - eine bessere Trackablity gegenüber einem Katheter mit einem konventionellen dreischichtigen HDPE-Innenschaft auf. Weiterhin ist die Kraftübertragung auf das distale Ende des Katheters gegenüber dem konventionellen dreischichtigen HDPE-Innenschaft deutlich erhöht. Dadurch können die Dimensionen (Außendurchmesser) des Innenschafts reduziert werden, ohne größere Einbußen in der Kraftübertragung zu erhalten. Außerdem wird das Profil von gecrimpten Stents von Stentdelivery RX-Kathetern reduziert.

Ferner hat sich herausgestellt, dass es leicht möglich ist, an die erfindungsgemäße Katheterschaftgestaltung weitere Bestandteile, wie z.B. distale Ballone, zuverlässig anzuschweißen. In dem im Beispiel (siehe unten) erstellten Katheter hielt der distale Ballonhals nach Anschweißung auf den erfindungsgemäß einzusetzenden ersten Schlauch (der als Innenschaft ausgestaltet war) Drücke von mindestens 24 bar aus. Bei diesem Druck platzte der Ballon. Dies entspricht einer ungefähren minimalen Spannung an der Verschweißung von 230 - 240 N/mm².

Die erhöhte Kraftübertragung (Pushability) gegenüber konventionellen RX-Kathetern mit dreischichtigen Innenschläuchen (HDPE-Tie-PA oder PEBA) verbessert die Einsetzbarkeit der erfindungsgemäßen Katheterschäfte bzw. der Medizinprodukte, die diese Schäfte umfassen.

Wenn der erfindungsgemäß einzusetzende erste Schlauch als distaler Außenschaft eingesetzt wird, sind niedrigere Reibeigenschaften gegeben, ohne dass ein zusätzliches aufwändiges und kostspieliges Coating erforderlich ist.

Durch die Strahlensterilisierbarkeit können die erfindungsgemäßen Katheterschäfte auch zum Beispiel für medikamentenbeschichte Katheter (Drug Eluting Stents or Drug Eluting Balloons) eingesetzt werden, bei denen das aufgebrachte Medikament nicht mittels Ethylenoxid sterilisiert werden kann.

Entsprechend dem oben Gesagten ist Teil der Erfindung die Verwendung eines ersten Schlauchs, wie oben definiert, zum Verschweißen mit einem zweiten Schlauch, wie ebenfalls oben definiert, zur Herstellung eines erfindungsgemäßen Katheterschaftes oder eines der erfindungsgemäßen Medizinprodukte.

Diese Verwendung ermöglicht die oben beschriebenen Vorteile.

Teil der Erfindung ist auch ein Verfahren zur Herstellung eines erfindungsgemäßen Katheterschaftes oder eines erfindungsgemäßen Medizinproduktes, umfassend die Schritte
a) Bereitstellen eines wie oben definierten ersten Schlauchs,
b) Bereitstellen eines wie oben definierten zweiten Schlauchs und
c) Verschweißen des ersten mit dem zweiten Schlauch.

Mittels dieses Verfahrens lassen sich die erfindungsgemäßen Katheterschäfte und die erfindungsgemäßen Medizinprodukte kostengünstig und zuverlässig herstellen.

Bevorzugt ist ein erfindungsgemäßes Verfahren, wobei das Verschweißen bei einer Temperatur von ≤ 200°C erfolgt.

Diese Verschweißbedingungen sind verhältnismäßig milde, so dass es ohne großen Aufwand möglich ist, die erfindungsgemäßen Produkte herzustellen.

Weiter bevorzugt ist ein erfindungsgemäßes Verfahren, wobei das Bereitstellen des ersten Schlauchs durch konventionelle Schlauchextrusion mit Abkühlung der geformten Schicht in Wasser erfolgt. Bevorzugt eingesetzt wird mit Maleinsäureanhydrid gepfropftes PVDF, welches wiederum bevorzugt in einem konventionellen Einschneckenextruder (Ø 12 - 30 mm / L/D 24-28) aufgeschmolzen und über ein Formgebungsteil zu einem Schlauch geformt wird, der in einem Wasserbad abgeschreckt wird.

Wie weiter oben beschrieben führt diese Vorgehensweise bei Vorliegen von vielen reaktiven Gruppen, insbesondere Maleinsäureanhydrid, dazu, dass eine erhöhte Reaktivität und somit eine verbesserte Verschweißbarkeit vorliegt, auch wenn die nicht-hydratisierten Anhydride reaktiver sind gegenüber Nucleophilen als die entstehenden Dicarbonsäuregruppen.

Weiter bevorzugt ist ein erfindungsgemäßes Verfahren, wobei das Verschweißen durch Wärmeeinwirkung für 3 - 20 Sekunden, weiter bevorzugt für 5 - 15 Sekunden, erfolgt.

Diese Verschweißdauer ist ausreichend, so dass ein verhältnismäßig schnelles Verfahren zur Herstellung der erfindungsgemäßen Gegenstände möglich ist.

Erfindungsgemäß weiter bevorzugt ist ein erfindungsgemäßes Verfahren, wobei das Verschweißen mittels Heizbacken, Laser, weißem Licht oder Vibration erfolgt.

Hierbei handelt es sich um gängige Mittel zum Verschweißen, die leicht handhabbar sind und für eine Reihe von Einsatzvarianten hervorragend verwendet werden können.

### Beispiele

Nachfolgend wird die Erfindung anhand von Beispielen und den dazugehörigen Figuren weiter erläutert.

Die Figuren stellen dar:
- Fig. 1: Die Pushability (Kraftübertragung) an coronaren Ballondilatationskathetern
- Fig. 2 a und 2b: Die Trackability (Reibungskräfte) an coronaren Ballondilatationskathetern in einem Aorten-Modell mit Teflon®-Schlauch als Reibungspartner
- Fig. 3: Eine schematische Zeichnung des Aufbaus eines Katheters mit erfindungsgemäßem Innenschaft
- Fig. 4: Eine schematische Zeichnung des Schweißens von Außen- und Innenschaft
- Fig. 5: Eine schematische Zeichnung einer distalen Ballonschweißung an einem erfindungsgemäßen Katheterschaft
- Fig. 6: Eine schematische Zeichnung der proximalen Schweißung eines distalen erfindungsgemäßen Katheters an eines Hypotube
- Fig. 7: Eine schematische Zeichnung einer proximalen Ballonschweißung

Es erfolgte die Extrusion des ersten Schlauchs im Regelfall aus einem chemisch modifizierten PVDF (z. B. Kynar ADX 1740-15 (PA) der Firma Arkema) auf einem konventionellen Einschneckenextruder (z.B. Ø 12 - 30 mm mit einem L/D-Verhältnis von ∼ 24).

### 1. Extrusion eines einschichtigen erfindungsgemäß einzusetzenden Schlauchs

Das Granulat Kynar ADX 1740-15 (PA) wurde in einem Presslufttrockner (Taupunkt ∼ -30°C) für 4 - 6 h bei 70 - 100°C getrocknet.

Das getrocknete Granulat wurde über die Einzugszone eines Einschneckenextruders (0 12 - 30 mm, L= 24 - 28 D) in den Extruder dosiert und bei den von Arkema vorgeschlagenen Temperaturen im Extruder aufgeschmolzen. Die Schmelze wurde über einen Extrusionskopf, bestehend aus einer Düse und einem Dorn, aus dem Extruder gefördert und der so geformte Schlauch auf die zu erzielende finale Schlauchdimension kalibriert, wobei eine Draw-Rate-Balance von ∼1.0 und ein Deep-Draw-Ratio von 8 - 10 benutzt wurde.

Der resultierende Schlauch wurde mittels Zugprüfung charakterisiert:

| | |
|---|---|
| Streckspannung: | 46N/mm2 |
| Streckdehnung: | 6% |
| Bruchspannung: | 100N/mm2 |
| Bruchdehnung: | 254% |
| E-Modul: | 1120 N/mm2 |

### 2. Innenschaftschlauch/Außenschlauch-Verschweißung

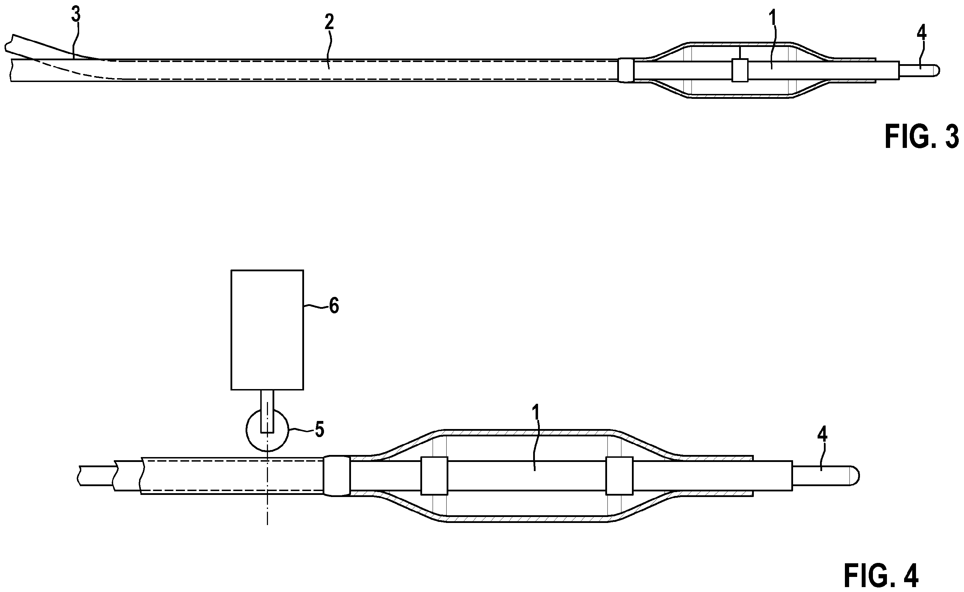

Die Fig. 3 stellt schematisch einen erfindungsgemäßen Katheterschaft (Katheter mit erfindungsgemäßem Innenschaft) dar. Dabei haben die Bezugszeichen folgende Bedeutung:
- 1: erster Schlauch (Innenschlauch)
- 2: zweiter Schlauch (Außenschlauch)
- 3: Führungsdraht Austrittstelle
- 4: Hilfsdraht

Nachdem der unter 1.) hergestellte erste Schlauch 1 über die Führungsdrahtaustrittstelle 3 des zweiten Schlauchs 2 in diesen eingeführt wurde, wurde in den ersten Schlauch 1 ein Hilfsdraht 4 eingeführt.

Fig. 4 stellt schematisch den Vorgang des Zusammenschweißens des ersten und des zweiten Schlauchs (Außen- und Innenschaft) dar. Hierbei haben die Bezugszeichen folgende Bedeutung:
- 1: erster Schlauch
- 4: Hilfsdraht
- 5: Si-Schlauch, um ein Verschmelzen des Außenschlauchs 2 mit der Schweißbacke 6 zu verhindern.
- 6: Schweißbacke

Der erste Schlauch 1 (Innenschaft) wurde mittels Schweißbacke 6 an den zweiten Schlauch 2 (Außenschaft) bei Schweißbackentemperaturen von 185 - 205°C während 5 - 15 Sekunden geschweißt.

### 3. Distale Ballonschweißung

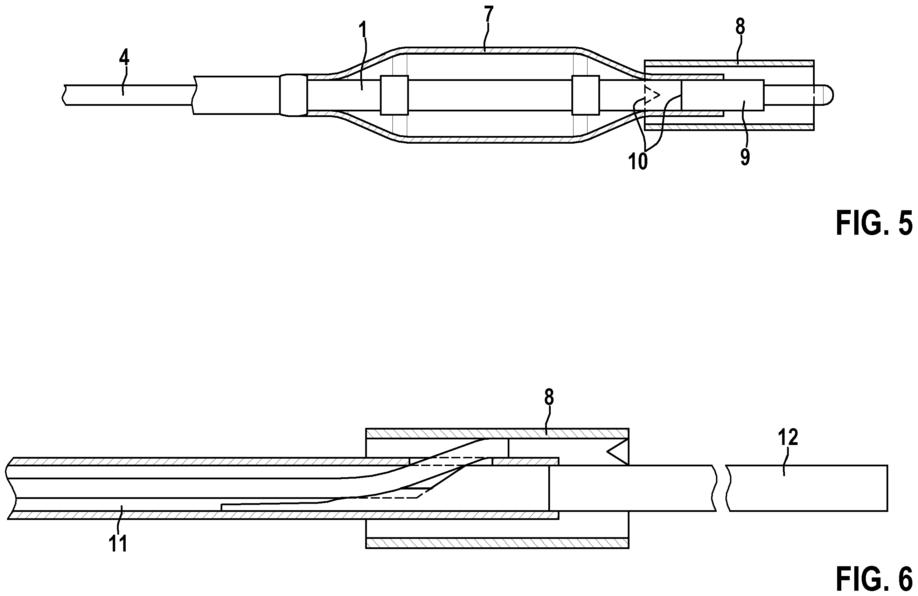

Die Fig. 5 stellt schematisch das Anschweißen eines distalen Ballons an den erfindungsgemäßen Katheterschaft dar. Hierbei haben die Bezugszeichen folgende Bedeutung:
- 1: erster Schlauch
- 4: Hilfsdraht
- 7: Ballon
- 8: Schrumpfschlauch
- 9: Spitze
- 10: bündige Anlagekante

In den ersten Schlauch 1 wurde ein geeigneter Hilfsdraht 4 eingeführt. Auf diesen Hilfsdraht wurde am distalen Ende des ersten Schlauchs 1 (Innenschaft) eine sogenannte Spitze 9 aus Polyamid positioniert. Über das distale Ende des ersten Schlauchs 1 und das proximale Ende der Spitze 9 wurde der distale Ballonhals positioniert. Über dem distalen Ballonhals wurde ein Schrumpfschlauch 8 positioniert. Der distale Ballonhals wurde mittels eines Lasers mit einer Leistung von 20 - 25 Watt innerhalb von 4 - 7 Sekunden auf den ersten Schlauch 1 und die Spitze 9 geschweißt, wobei sich der zu schweißende Bereich in axialer Richtung mit 1.500 - 2.500 U/min drehte.

### 4. Führungsdrahtaustrittstellen-Schweißung

Das unter 3.) gefertigte distale Teil des Katheters wurde nun mit dem proximalen Teil eines Hypotubes verbunden. Dies wird schematisch in der Fig. 6 dargestellt. Dabei bedeuten die Bezugszeichen:
- 8: Schrumpfschlauch
- 11: Vorderteil des Katheters
- 12: Hypotube

Hierzu wurde der zweite Schlauch 2 mit dem ersten Schlauch 1 auf den mit Polyamid ummantelten Hypotube mittels eines Schrumpfschlauchs geschoben. Der zweite Schlauch 2 wurde dann mit Schweißbacken 8 - 20 Sekunden bei 215 - 235°C geschweißt. Gleichzeitig wurde auch der erste Schlauch 1 an den zweiten Schlauch 2 geschweißt.

### 5. Proximale Ballonschweißung

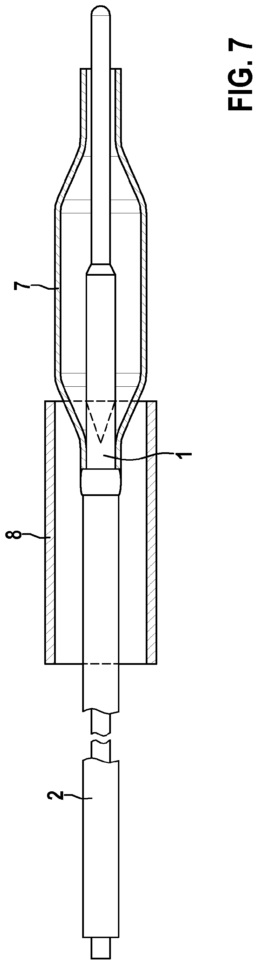

Fig. 7 stellt die proximale Ballonschweißung schematisch dar. Hierbei bedeuten die Bezugszeichen:
- 1: erster Schlauch
- 2: zweiter Schlauch
- 7: Ballon
- 8: Schrumpfschlauch

In den zweiten Schlauch 2 wurde ein passender Draht eingeführt und der proximale Ballonhals auf dem distalen Ende des zweiten Schlauchs 2 (Außenschaft) positioniert. Über die zu schweißende Stelle wurde ein Schrumpfschlauch 25 positioniert. Mittels eines Lasers wurde für 1 bis 4 Sekunden mit einer Energie von 15 bis 25 Watt ein Ballonhals auf den zweiten Schlauch 2 geschweißt. Danach wurde der Schrumpfschlauch entfernt.

Alle oben durchgeführten Schweißungen wurden mittels einer Druckprüfung geprüft. Dabei versagte keine der beschriebenen Schweißungen, wobei diese mindestens Drücken von 24 bar ausgesetzt wurden.

## Patentansprüche

1. Katheterschaft, umfassend
(i) einen ersten Schlauch (1) aus einem extrudierbaren fluorierten Copolymer, das reaktive Gruppen umfasst, und
(ii) einen mit dem ersten Schlauch (1) verschweißten zweiten Schlauch (2) aus einem polymeren Material, wobei das polymere Material des zweiten Schlauchs (2) bei einer Temperatur von ≤ 200°C mit dem ersten Schlauch (1) verschweißbar ist.

2. Katheterschaft nach Anspruch 1, wobei das fluorierte Copolymer ein Copolymer ist auf Basis von einem Polymer, ausgewählt aus der Gruppe bestehend aus ETFE, C-ETFE, PFA, FEP, PCTFE, PVF und PVDF.

3. Katheterschaft nach Anspruch 1 oder 2, wobei die reaktiven Gruppen ausgewählt sind aus der Gruppe bestehend aus Carbonsäure, Carbonsäurechlorid, Amid, Carbonsäureanhydrid, Ester, Lacton, Lactam, Nitril und Thioester.

4. Katheterschaft nach einem der vorangehenden Ansprüche, wobei das polymere Material des zweiten Schlauchs (2) ausgewählt ist aus der Gruppe bestehend aus Polyamid, PEBA, Polyester oder TPU.

5. Katheterschaft nach einem der vorangehenden Ansprüche, wobei der erste Schlauch (1) aus mit Maleinsäureanhydrid als reaktiver Gruppe gepfropftem PVDF besteht und der zweite Schlauch (2) aus Polyamid 12 oder PEBA besteht.

6. Katheterschaft nach einem der vorangehenden Ansprüche, wobei die Schläuche (1,2) bei einem Druck von 20 bar für ≥ 1 min unverändert miteinander verschweißt bleiben.

7. Medizinprodukt, umfassend einen Katheterschaft nach einem der vorangehenden Ansprüche.

8. Medizinprodukt nach Anspruch 7, ausgewählt aus der Gruppe bestehend aus Katheterstenteinlegungsvorrichtung oder Ballondilationskatheter, bevorzugt in RX-(Rapid Exchange) oder "Over the Wire"-Ausführung.

9. Verwendung eines ersten Schlauchs (1), wie in einem der Ansprüche 1 bis 3 oder 5 definiert, zum Verschweißen mit einem zweiten Schlauch (2), wie in einem der Ansprüche 1, 4 oder 5 definiert, zur Herstellung eines Katheterschaftes nach einem der Ansprüche 1 bis 6 oder eines Medizinproduktes nach einem der Ansprüche 7 oder 8.

10. Verfahren zur Herstellung eines Katheterschaftes nach einem der Ansprüche 1 bis 6 oder eines Medizinproduktes nach einem der Ansprüche 7 oder 8, umfassend die Schritte:
a) Bereitstellen eines ersten Schlauches (1), wie in einem der Ansprüche 1 bis 3 oder 5 definiert,
b) Bereitstellen eines zweiten Schlauches (2), wie in einem der Ansprüche 1, 4 oder 5 definiert, und
c) Verschweißen des ersten (1) und des zweiten Schlauches (2).

11. Verfahren nach Anspruch 10, wobei das Verschweißen bei einer Temperatur von ≤ 200°C erfolgt.

12. Verfahren nach Anspruch 10 oder 11, wobei das Bereitstellen in Schritt a) durch konventionelle Schlauchextrusion mit Abkühlung des geformten Schlauches in Wasser erfolgt.

13. Verfahren nach einem der Ansprüche 10 bis 12, wobei das Verschweißen durch Wärmeeinwirkung für 3 bis 20 Sekunden erfolgt.

14. Verfahren nach einem der Ansprüche 10 bis 13, wobei das Verschweißen mittels Heizbacken, Licht oder Vibration erfolgt.

15. Schlauch (1) aus mit Maleinsäureanhydrid als reaktiver Gruppe gepfropftem PVDF als Bestandteil eines Katheterschaftes nach einem der Ansprüche 1 bis 6 oder eines Medizinproduktes nach einem der Ansprüche 7 oder 8.

## Claims

1. A catheter shaft comprising
(i) a first tube (1) made of an extrudable fluorinated copolymer that has reactive groups, and
(ii) a second tube (2) made of a polymeric material, which is welded to the first tube (1), wherein the polymeric material of the second tube (2) can be welded to the first tube (1) at a temperature ≤ 200°C.

2. The catheter shaft according to claim 1, wherein the fluorinated copolymer is a copolymer on the basis of a polymer selected from the group comprising ETFE, C-ETFE, PFA, FEP, PCTFE, PVF and PVDF.

3. The catheter shaft according to claim 1 or 2, wherein the reactive groups are selected from the group comprising carbonic acid, carbonic acid chloride, amide, carbonic acid anhydride, ester, lactone, lactam, nitrile and thioester.

4. The catheter shaft according to any one of the preceding claims, wherein the polymeric material of the second tube (2) is selected from the group comprising polyamide, PEBA, polyester or TPU.

5. The catheter shaft according to any one of the preceding claims, wherein the first tube (1) is made of PVDF grafted with maleic acid anhydride as the reactive group, and the second tube (2) is made of polyamide 12 or PEBA.

6. The catheter shaft according to any one of the preceding claims, wherein the tubes (1,2) remain welded to one another, unchanged, at a pressure of 20 bar for ≥ 1 min.

7. A medical device comprising a catheter shaft according to any one of the preceding claims.

8. The medical device according to claim 7, selected from the group comprising a catheter stent insertion device or a balloon dilation catheter, in particular in an RX (rapid exchange) or "over the wire" design.

9. The use of a first tube (1) as defined in any one of the claims 1 to 3 or 5 for welding to a second tube (2) as defined in any one of the claims 1, 4 or 5, to produce a catheter shaft according to any one of the claims 1 to 6 or a medical device according to any one of the claims 7 or 8.

10. A method for manufacturing a catheter shaft according to any one of the claims 1 to 6 or a medical device according to any one of the claims 7 or 8, comprising the steps:
a) provide a first tube (1) as defined in any one of the claims 1 to 3 or 5,
b) provide a second tube (2) as defined in any one of the claims 1, 4 or 5, and
c) weld the first tube (1) and the second tube (2).

11. A method according to claim 10, wherein welding takes place at a temperature ≤ 200°C.

12. The method according to claim 10 or 11, wherein the provision in step a) takes place by conventional tube extrusion with cooling of the formed tube in water.

13. The method according to any one of the claims 10 to 12, wherein welding takes place by the effect of heat for 3 to 20 seconds.

14. The method according to any one of the claims 10 to 13, wherein welding takes place by way of heating jaws, light or vibration.

15. A tube (1) made of PVDF grafted with maleic acid anhydride as the reactive group, as a component of a catheter shaft according to any one of the claims 1 to 6 or a medical device according to any one of the claims 7 or 8.

## Revendications

1. Tige de cathéter, comprenant
(i) une première tubulure (1) à base d'un copolymère fluoré extrudable, qui comprend des groupes réactifs, et
(ii) une deuxième tubulure (2) soudée avec la première tubulure (1) à base d'un matériau polymère, où le matériau polymère de la deuxième tubulure (2) peut être soudé avec la première tubulure (1) à une température ≤ 200 °C.

2. Tige de cathéter selon la revendication 1, dans lequel le copolymère fluoré est un copolymère à base d'un polymère choisi dans le groupe constitué de l'éthylène tétrafluoroéthylène ETFE, de l'ETFE-C, de perfluoroalcoxy PFA, d'éthylène propylène fluoré FEP, de polychlorotrifluoroéthylène PCTFE, de polyfluorure de vinyle PVF et de polyfluorure de vinylidène PVDF.

3. Tige de cathéter selon la revendication 1 ou 2, dans laquelle les groupes réactifs sont choisis dans le groupe constitué de l'acide carbonique, du chlorure d'acide carbonique, d'un amide, de l'anhydride de l'acide carbonique, d'un ester, d'une lactone, d'un lactame, d'un nitrile et d'un thioester.

4. Tige de cathéter selon l'une des revendications précédentes, dans laquelle le matériau polymère de la deuxième tubulure (2) est choisi dans le groupe constitué de polyamide, de copolymère bloc éther amide PEBA, de polyester ou de polyuréthane thermoplastique TPU.

5. Tige de cathéter selon l'une des revendications précédentes, dans laquelle la première tubulure (1) est constituée d'un PVDF greffé avec l'anhydride d'acide maléique en tant que groupe réactif et la deuxième tubulure (2) est constituée de polyamide 12 ou de PEBA.

6. Tige de cathéter selon l'une des revendications précédentes, dans laquelle les tubulures (1, 2) restent soudées ensemble à l'état inchangé pour ≥ 1 minute à une pression de 20 bar.

7. Produit médical comprenant une tige de cathéter selon l'une des revendications précédentes.

8. Produit médical selon la revendication 7, choisi dans le groupe constitué d'un dispositif de mise en place d'un cathéter de stent ou d'un cathéter de ballonnet de dilatation, de préférence dans une version RX (Rapid Exchange) ou « Over the Wire ».

9. Utilisation d'une première tubulure (1) telle que définie dans l'une des revendications 1 à 3 ou 5 pour la soudure avec une deuxième tubulure (2), telle que définie dans l'une des revendications 1, 4 ou 5, pour la fabrication d'une tige de cathéter selon l'une des revendications 1 à 6 ou d'un produit médical selon l'une des revendications 7 ou 8.

10. Procédé de fabrication d'une tige de cathéter selon l'une des revendications 1 à 6 ou d'un produit médical selon l'une des revendications 7 ou 8, comprenant les étapes :
a) mise au point d'une première tubulure (1), telle que définie dans l'une des revendications 1 à 3 ou 5,
b) mise au point d'une deuxième tubulure (2) telle que définie dans l'une des revendications 1, 4 ou 5,
c) soudure de la première (1) et de la deuxième tubulure (2).

11. Procédé selon la revendication 10, dans lequel la soudure a lieu à une température ≤ 200 °C.

12. Procédé selon la revendication 10 ou 11, dans lequel la mise au point dans l'étape a) a lieu par une extrusion de la tubulure conventionnelle avec un refroidissement de la tubulure formée dans l'eau.

13. Procédé selon l'une des revendications 10 à 12, dans lequel la soudure a lieu par une influence de la chaleur pendant 3 à 20 secondes.

14. Procédé selon l'une des revendications 10 à 13, dans lequel la soudure a lieu au moyen d'une mâchoire chauffante, de lumière ou d'une vibration.

15. Tubulure (1) à base de PVDF greffé avec de l'anhydride d'acide maléique en tant que groupe réactif servant de composant d'une tige de cathéter selon l'une des revendications 1 à 6 ou d'un produit médical selon l'une des revendications 7 ou 8.
